# EUROPÄISCHE PATENTSCHRIFT

(11) **EP 2 490 643 B1**
(45) Veröffentlichungstag und Bekanntmachung des Hinweises auf die Patenterteilung: **24.09.2014**
(21) Anmeldenummer: 10770719.2
(22) Anmeldetag: 14.10.2010
(51) Int. Cl.: A61H 1/02, A61H 1/00, A61G 13/00, A61G 13/12, A61F 5/37

(54) **BEHANDLUNGS- UND/ODER THERAPIEUNTERLAGE**
TREATMENT AND/OR THERAPY SUPPORT
BASE DE TRAITEMENT ET/OU DE THÉRAPIE

(30) Priorität: 21.10.2009 DE 102009050183
(43) Veröffentlichungstag der Anmeldung: 29.08.2012
(73) Patentinhaber: Bordas, Robert, 97490 Poppenhausen / Maibach (DE)
(72) Erfinder: Bordas, Robert, 97490 Poppenhausen / Maibach (DE)
(74) Vertreter: Gosdin, Michael
(86) Internationale Anmeldenummer: PCT/EP2010/006293
(87) Internationale Veröffentlichungsnummer: WO 2011/047802

(56) Entgegenhaltungen:
- WO-A1-2008/146429
- DE-A1- 3 126 071
- US-A- 4 166 459
- US-A- 5 713 816
- US-A- 5 820 573
- US-A1- 2009 229 048
- US-B1- 6 446 288

## Beschreibung

Die Erfindung betrifft eine Behandlungs- und/oder Therapieunterlage für die Behandlung und/oder Therapie, insbesondere für die Mobilisation, eines Teils der Wirbelsäule, insbesondere der Halswirbelsäule, des Menschen, wobei die Unterlage einen ersten Auflagebereich für den Kopf und einen zweiten Bereich aufweist.

Eine solche Unterlage ist beispielsweise aus der US 5 713 816 A und aus der GB 2 389 792 B bekannt. Die dort beschriebene Unterlage ist im wesentlichen symmetrisch zu einer Mittenachse ausgebildet, die eine Längsachse umfasst, die entlang des Körpers der zu behandelnden Person verläuft. Im Bereich der Mittenebene sind Ausnehmungen eingebracht, so dass insbesondere Wirbel der zu behandelnden Person nicht an vorspringenden Bereichen der Unterlage aufliegen.

Eine ähnliche Lösung zeigt die DE 10 2005 026 107 A1**.** Hier ist eine Wirbelsäulen-Therapieunterlage beschrieben, die in analoger Weise zu der vorstehend diskutierten Lösung im Bereich der Mittenebene Ausnehmungen aufweist, um eine Druckbelastung der Wirbel zu verhindern.

Auch die DE 31 26 071 A1 und die DE 295 19 551 U1 zeigen ähnliche Lösungen, wobei wiederum im Bereich einer Mittenebene Ausnehmungen vorgesehen sind, so dass die Wirbel der zu behandelnden Person nicht mit Druckkräften beaufschlagt werden. Weitere ähnliche Lösungen offenbaren die die US 2009/229048 A1**,** die US 5 820 573 A**,** die US 4 166 459 A**,** die WO 2008/146429 A1 und die US 6 446 288 B1**.**

Bei der Behandlung von Verspannungen im Bereich des Suboccibitalraums, d. h. des Bereichs unmittelbar unterhalb des Kopfs bzw. Hinterhaupts (Occiput) im Übergangsbereich zur Halswirbelsäule, erweisen sich die vorbekannten Behandlungs- bzw. Therapieunterlagen als nicht sehr hilfreich. Hier muss zumeist manuell mittels der Fingerspitzen ein Druck am Übergang des ersten Halswirbels (Atlas) zum Hinterkopf (Occiput) aufgebaut werden, um das Gewebe zu lockern.

Allerdings ist dieser Übergangsbereich, d. h. namentlich der Abstand zwischen dem Occiput zum Atlas, so klein, dass mitunter der Eingriff des Chiropraktikers schwierig ist.

Der Erfindung liegt daher die **Aufgabe** zugrunde, eine Behandlungs- und/oder Therapieunterlage der eingangs genannten Art so fortzubilden, dass es in verbesserter Weise möglich ist, den Bereich des Subocciput zu erreichen und auf ihn eine dehnende Kraft auszuüben, um eine Lockerung des Gewebes zu erreichen und so einen verbesserten Behandlungseffekt zu erzielen. Die Unterlage soll dabei sowohl für die Behandlung durch den Chiropraktiker als auch zur Selbstbehandlung geeignet sein.

Die **Lösung** dieser Aufgabe durch die Erfindung ist dadurch gekennzeichnet, dass zwischen dem ersten Auflagebereich und dem zweiten Bereich eine Erhebung zur Auflage des Subocciput-Bereichs angeordnet ist, wobei die Erhebung im Bereich einer Mittenebene liegt, die vertikal angeordnet ist und eine Längsachse der Unterlage beinhaltet, wobei sich die Erhebung, in Richtung der Längsachse gesehen, horizontal erstreckt, wobei sich die Erhebung, in Richtung der Längsachse gesehen, symmetrisch zur Mittenebene erstreckt, wobei seitlich der Längsachse neben der Erhebung Vertiefungen angeordnet sind, wobei die Erhebung eine nach oben gerichtete Ausdehnungsrichtung aufweist, die bei bestimmungsgemäßem Gebrauch der Unterlage zu einer vertikalen Richtung einen spitzen Winkel einschließt, der zwischen 5° und 45°, vorzugsweise zwischen 20° und 30°, beträgt, und wobei die unter spitzem Winkel zur Vertikalen sich erstreckende Erhebung mit ihrer in Richtung der Längsachse gerichteten Komponente zum ersten Bereich weist.

Die Erhebung erstreckt sich dabei bevorzugt über eine Breite zwischen 1 cm und 7 cm.

Die Unterlage ist dabei bevorzugt symmetrisch zur vertikal angeordneten Mittenebene ausgebildet. Diese Längsachse entspricht dem (begradigten) Verlauf der Wirbelsäule der die Unterlage benutzenden Person.

Seitlich der Längsachse können neben der Erhebung Vertiefungen angeordnet sein. Dabei ist bevorzugt vorgesehen, dass weiterhin seitlich der Längsachse neben den Vertiefungen im von der Erhebung entfernten Endbereich der Vertiefungen je eine Seitenstütze angeordnet ist. Die Seitenstützen können dabei die Erhebung um einen Überstand überragen, wobei dieser Überstand bevorzugt zwischen 0,5 cm und 2 cm beträgt.

Die unter spitzem Winkel zur Vertikalen sich erstreckende Erhebung weist - wie erläutert - mit ihrer in Richtung der Längsachse gerichteten Komponente zum ersten Bereich, d. h. der auf der Erhebung aufliegende Bereich des Subocciput wird relativ zu der die Unterlage benutzenden Person aufgrund des Winkels nach oben gedrückt.

Die Erhebung weist dabei in Richtung der Längsachse in ihrem oberen Endbereich vorzugsweise eine Ausdehnung (d. h. Breite) zwischen 0,3 cm und 2 cm auf. Sie kann in einem Schnitt entlang einer Ebene, die die Richtung der Längsachse und die Vertikale umfasst (d. h. in einem Schnitt entlang der Symmetrieebene), in ihrem oberen Endbereich eine abgerundete Form aufweisen, insbesondere mit einem Radius zwischen 0,15 bis 1 cm.

Die Unterlage kann aus Kunststoff gefertigt werden, wobei beispielsweise Polyurethan als ein mögliches Material bevorzugt ist.

Mit dem Erfindungsvorschlag wird es möglich, den Bereich des Suboccibitalraums bei der Wirbelsäulen-Therapie verbessert zu erreichen und eine definierte dosierte Kraft auf den Bereich auszuüben.

Durch die winkelige Anstellung der Erhebung kann eine Kraftwirkung auf den Bereich des Subocciputs ausgeübt werden, der zu einer Dehnung des Gewebes oberhalb des Atlas führt. Durch die Ausbildung und die Abmessungen der Erhebung kann dieser Effekt gezielt gesteuert werden.

Hierfür wird auf die gezielt mittige, d. h. auf der Symmetrieebene liegende Unterstützung durch die Erhebung zurückgegriffen, die im Stand der Technik gerade vermieden wird. Vorteilhaft ist auch die seitliche Unterstützung durch die genannten Seitenstützen.

Diese Unterstützung durch die Erhebung ist im genannten Bereich auch nicht problematisch, weil der sich dort befindliche Halswirbel (Atlas) keinen Dornfortsatz aufweist. Vielmehr kann gezielt ein Druck auf das Gewebe aufgebaut werden.

Demgemäß muss für die genannte Behandlung nicht mehr nur auf die rein manuelle Therapie zurückgegriffen werden, wie es bislang der Fall war. Somit eröffnet sich auch die Möglichkeit der Selbstbehandlung ohne Therapeuten.

Im Unterschied zu manch anderen vorbekannten Lösungen unterstützt die Erhebung den Patienten im Bereich einer Mitten- bzw. Symmetrieebene. Bei vorbekannten Lösungen ist gerade dieser Bereich zumeist mit einer Ausnehmung versehen, so dass hierdurch gerade keine effiziente Behandlung erfolgen kann.

In der Zeichnung ist ein Ausführungsbeispiel der Erfindung dargestellt. Es zeigen:
- Fig. 1: in perspektivischer Ansicht eine Behandlungs- und/oder Therapieunterlage,
- Fig. 2: die Behandlungs- und/oder Therapieunterlage nach Fig. 1 im Schnitt C-D gemäß Fig. 3 und
- Fig. 3: die Behandlungs- und/oder Therapieunterlage nach Fig. 1 im Schnitt A-B gemäß Fig. 2.

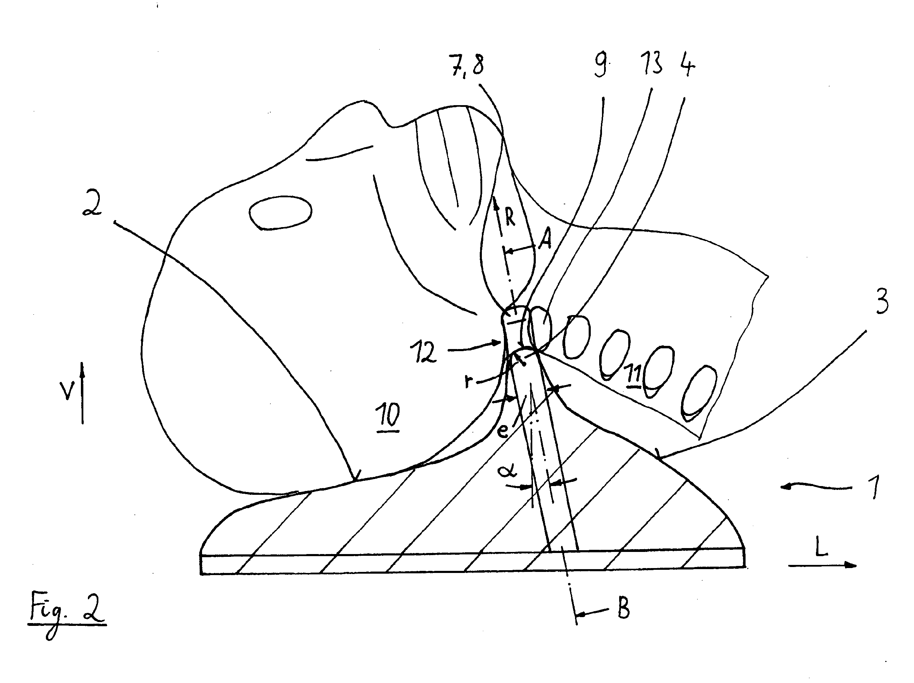

In den Figuren ist eine Behandlungs- und Therapieunterlage 1 skizziert, die bestimmungsgemäß vorgesehen ist, um unter den Hinterkopfbereich und Halswirbelbereich einer zu behandelnden Person untergelegt zu werden. In Fig. 2 ist angedeutet, wie der Kopf- und Halswirbelbereich der zu behandelnden Person zu liegen kommt.

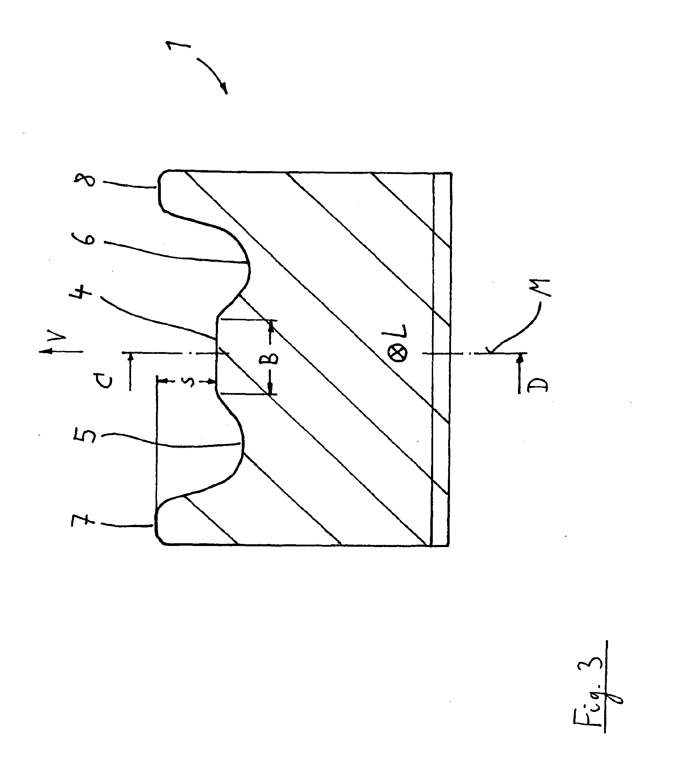

Die Unterlage 1 ist entlang einer Ebene symmetrisch ausgebildet, die in vertikale Richtung V und in Richtung einer Längsachse L verläuft. Die Längsachse entspricht der (begradigt gedachten) Längserstreckung der Wirbelsäule der zu behandelnden Person. Eine sich hierbei ergebende Mittenebene M ist in Fig. 3 markiert.

Die Unterlage 1 hat zwei Bereiche 2 und 3, nämlich einen ersten Auflagebereich 2, der für die Auflage des Kopfs 10 der zu behandelnden Person bestimmt ist, sowie einen zweiten Bereich 3, der unterhalb des Halsbereichs 11 zu liegen kommt, s. Fig. 2. Der Bereich 3 kann dabei auch sehr klein ausgebildet sein und fast senkrecht abfallen; er dient letztlich nur der Stabilisierung der Unterlage 1.

In Fig. 2 ist auch der Suboccipitalbereich 12 markiert, d. h. der Bereich unterhalb des Occiput (Hinterhaupt) und dem ersten Wirbel 13 der Halswirbelsäule, d. h. dem Atlas.

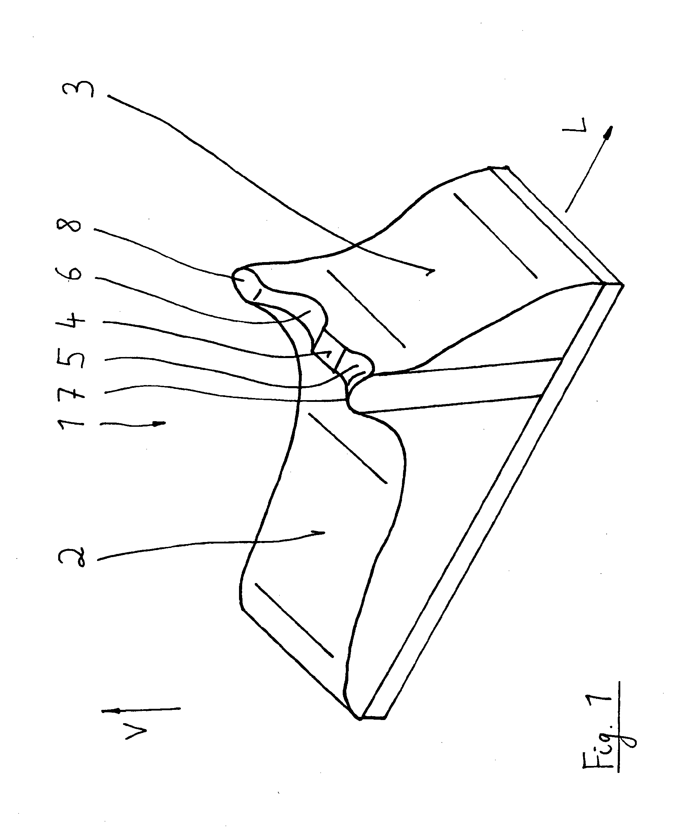

In der Zusammenschau der Figuren 1 bis 3 ergibt sich folgendes:
In Richtung der Längsachse L ist zwischen dem ersten Auflagebereich 2 und dem zweiten Bereich 3 ein Auflagebereich vorgesehen, der gezielt den Bereich des Subocciput unterstützen soll. Dieser Bereich wird im wesentlichen durch eine Erhebung 4 gebildet, die sich mittig, also in der genannten Symmetrieebene vertikal nach oben erstreckt. Bei der Erhebung 4 handelt es sich um einen Bereich, der sich in eine Ausdehnungsrichtung R erstreckt (s. Fig. 2), die unter einem spitzen Winkel α zur Vertikalen V verläuft. Der Winkel α beträgt bevorzugt zwischen 20° und 30°.

Die Breite B der Erhebung ist mit B angegeben; dieser Wert liegt bevorzugt zwischen 1 cm und 7 cm.

Seitlich flankiert wird die Erhebung 4 von zwei Vertiefungen 5 und 6, was zur Folge hat, dass der Bereich des Subocciput definiert und alleine von der Erhebung 4 unterstützt auf derselben aufliegt. In den Seitenbereichen der Unterlage 1 befinden sich allerdings Seitenstützen 7 und 8, die den Bereich des Atlas seitlich abstützen. Es ist zu erkennen, dass die Seitenstützen 7, 8, in vertikale Richtung V gesehen, die Erhebung 4 überragen, nämlich um einen Überstand s, der im Bereich zwischen 0,3 und 5 cm liegen kann.

In der Ansicht gemäß Fig. 2 ist weiter zu erkennen, dass der obere Endbereich der Erhebung 4 abgerundet ausgebildet ist. Hier ist eine Abrundung 9 vorgesehen, die einen Radius r haben kann, der im Bereich zwischen 0,25 und 1,5 cm liegen kann.

Aus dieser Darstellung ist auch ersichtlich, dass die Erhebung 4 in Richtung der Längsachse L betrachtet (wenn man einmal von dem kleinen Winkel α absieht) eine Ausdehnung e aufweist, die bevorzugt im Bereich zwischen 0,3 und 2 cm liegt.

Durch die Wahl der genannten geometrischen Parameter wird es möglich, einen gezielten Druck im Bereich 12 des Subocciput aufzubauen, wobei ersichtlich ist, dass infolge der winkeligen Ausrichtung der Erhebung 4 eine Kraftwirkung erzielt wird, die den Atlas bzw. Suboccipitalbereich "nach oben" drängt, so dass der Suboccipitalbereich 12 gelockert werden kann.

### Bezugszeichenliste:

- 1: Behandlungs- und/oder Therapieunterlage
- 2: erster Auflagebereich
- 3: zweiter Auflagebereich
- 4: Erhebung
- 5: Vertiefung
- 6: Vertiefung
- 7: Seitenstütze
- 8: Seitenstütze
- 9: Abrundung
- 10: Kopf
- 11: Halsbereich
- 12: Suboccipitalbereich
- 13: Atlas

- L: Längsachse
- M: Mittenebene
- B: Breite
- s: Überstand
- e: Ausdehnung
- R: Ausdehnungsrichtung
- r: Radius
- V: vertikale Richtung
- α: Winkel

## Patentansprüche

1. Behandlungs- und/oder Therapieunterlage (1) für die Behandlung und/oder Therapie, insbesondere für die Mobilisation, eines Teils der Wirbelsäule, insbesondere der Halswirbelsäule, des Menschen, wobei die Unterlage (1) einen ersten Auflagebereich (2) für den Kopf (10) und einen zweiten Bereich (3) aufweist,
**dadurch gekennzeichnet,**
**dass** zwischen dem ersten Auflagebereich (2) und dem zweiten Bereich (3) eine Erhebung (4) zur Auflage des Suboccipitalbereichs (12) angeordnet ist,
wobei die Erhebung (4) im Bereich einer Mittenebene liegt, die vertikal angeordnet ist und eine Längsachse (L) der Unterlage (1) beinhaltet,
wobei sich die Erhebung (4), in Richtung der Längsachse (L) gesehen, horizontal erstreckt,
wobei sich die Erhebung (4), in Richtung der Längsachse (L) gesehen, symmetrisch zur Mittenebene erstreckt,
wobei seitlich der Längsachse (L) neben der Erhebung (4) Vertiefungen (5, 6) angeordnet sind,
wobei die Erhebung (4) eine nach oben gerichtete Ausdehnungsrichtung (R) aufweist, die bei bestimmungsgemäßem Gebrauch der Unterlage (1) zu einer vertikalen Richtung (V) einen spitzen Winkel (α) einschließt, der zwischen 5° und 45° beträgt, und
wobei die unter spitzem Winkel (α) zur Vertikalen (V) sich erstreckende Erhebung (4) mit ihrer in Richtung der Längsachse (L) gerichteten Komponente zum ersten Bereich (2) weist.

2. Behandlungs- und/oder Therapieunterlage nach Anspruch 1, **dadurch gekennzeichnet, dass** der Winkel (α) zwischen 20° und 30° beträgt.

3. Behandlungs- und/oder Therapieunterlage nach Anspruch 1 oder 2, **dadurch gekennzeichnet, dass** sich die Erhebung (4) über eine Breite (B) zwischen 1 cm und 7 cm erstreckt.

4. Behandlungs- und/oder Therapieunterlage nach einem der Ansprüche 1 bis 3, **dadurch gekennzeichnet, dass** sie symmetrisch zu der vertikal angeordneten Mittenebene ausgebildet ist.

5. Behandlungs- und/oder Therapieunterlage nach einem der Ansprüche 1 bis 4, **dadurch gekennzeichnet, dass** seitlich der Längsachse (L) neben den Vertiefungen (5, 6) im von der Erhebung (4) entfernten Endbereich der Vertiefungen (5, 6) je eine Seitenstütze (7, 8) angeordnet ist.

6. Behandlungs- und/oder Therapieunterlage nach Anspruch 5, **dadurch gekennzeichnet, dass** die Seitenstützen (7, 8) die Erhebung (4) um einen Überstand (s) überragen, wobei der Überstand (s) bevorzugt zwischen 0,5 cm und 2 cm beträgt.

7. Behandlungs- und/oder Therapieunterlage nach einem der Ansprüche 1 bis 6, **dadurch gekennzeichnet, dass** die Erhebung (4) in Richtung der Längsachse (L) in ihrem oberen Endbereich eine Ausdehnung (e) zwischen 0,3 cm und 2 cm aufweist.

8. Behandlungs- und/oder Therapieunterlage nach einem der Ansprüche 1 bis 7, **dadurch gekennzeichnet, dass** die Erhebung (4) in einem Schnitt entlang einer Ebene, die die Richtung der Längsachse (L) und die Vertikale (V) umfasst, in ihrem oberen Endbereich eine abgerundete Form (9) aufweist, insbesondere mit einem Radius (r) zwischen 0,15 bis 1 cm.

## Claims

1. Support for treatment and/or therapy (1), especially for the mobilization of a part of the human spine, especially the cervical spine, with a first area of support (2) for the head (10) and a second area of support (3),
**characterized in that**
an elevation (4) is located between the first area of support (2) and the second area of support (3) intended for the rest of the suboccipital area (12),
wherein the elevation (4) is located in a middle plain, which is aligned vertically and comprises a longitudinal axis (L) of the support (1),
wherein the elevation (4) extends horizontally when looking in the direction of the longitudinal axis (L),
wherein the elevation (4) extends symmetrically to the middle plain when looking in direction of the longitudinal axis (L),
wherein recesses (5, 6) are located besides the elevation (4) laterally to the longitudinal axis (L),
wherein the elevation (4) comprises an upward direction of expansion (R), which encloses at intended use of the support (1) an acute angle (α), to the vertical direction (V) which lies between 5° and 45°, and
wherein the elevation (4) points into the direction of the first area of support (2) which elevation (4) extends under the acute angle (α) to the vertical direction (V) with its component which is directed along the longitudinal axis (L).

2. Support for treatment and/or therapy (1) according to claim 1, **characterized in that** the angle (α) is between 20° and 30°.

3. Support for treatment and/or therapy (1) according to claim 1 or 2, **characterized in that** the elevation (4) extends along a width (B) between 1 cm and 7 cm.

4. Support for treatment and/or therapy (1) according to one of claims 1 to 3, **characterized in that** it is symmetrical to the vertical middle plain.

5. Support for treatment and/or therapy (1) according to one of claims 1 to 4, **characterized in that** laterally from the longitudinal axis (L) in each case a lateral support (7, 8) is arranged beside the recesses (5, 6) in the end region of the recesses (5, 6) remote from the elevation (4).

6. Support for treatment and/or therapy (1) according to claim 5, **characterized in that** the lateral supports (7, 8) exceed the elevation (4) by a protrusion (s), wherein the protrusion (s) lies preferably between 0.5 cm and 2 cm.

7. Support for treatment and/or therapy (1) according to one of claims 1 to 6, **characterized in that** the elevation (4) has a thickness (e) in its upper end region in the direction of the longitudinal axis (L), which lies between 0.3 to 2 cm.

8. Support for treatment and/or therapy (1) according to one of claims 1 to 7 **characterized in that** the elevation (4) comprises a rounded shape (9) in its upper end region in a section along a plain, which includes the longitudinal axis (L) and the vertical axis (V), especially with a radius (r) of between 0.15 to 1 cm.

## Revendications

1. Bloc de traitement et / ou de thérapie (1) pour le traitement et / ou la thérapie, en particulier pour la mobilisation d'une partie de la colonne vertébrale, en particulier des vertèbres cervicales, de l'être humain, dans lequel le support (1) comporte une première zone de repos (2) pour la tête (10) et qu'une seconde zone (3),
**caractérisée en ce**
**qu'**entre la première zone de repos (2) et la seconde zone (3) une élévation (4) pour soutenir la région suboccipitale (12) est prévue, cette élévation (4) étant dans la région d'un plan central disposé verticalement et comportant un axe longitudinal (L) à la base (1), l'élévation (4), vue dans la direction de l'axe longitudinal (L) s'étendant horizontalement l'élévation (4), vue dans la direction de l'axe longitudinal (L) s'étendant symétriquement par rapport au plan central, des creux (5,6) étant agencés le long de l'axe longitudinal (L) à côté de l'élévation (4), l'élévation (4) s'étendant vers le haut (R), ce qui, dans des conditions normales d'utilisation de la base (1) inclut un angle aigu (α) compris entre 5 degrés et 45 degrés, et les composants de l'élévation (4) en angle aigu (α) avec la verticale (V) qui s'étendent dans la direction de l'axe longitudinal (L) étant dirigés vers la première zone (2).

2. Bloc de traitement et / ou de thérapie selon l'exigence 1, **caractérisé en ce que** l'angle (α) est compris entre 20 degrés et 30 degrés.

3. Bloc de traitement et / ou de thérapie selon l'exigence 1 ou 2, **caractérisé en ce que** l'élévation (4) s'étend sur une largeur (B) comprise entre 1 cm et 7 cm.

4. Bloc de traitement et / ou de thérapie selon l'une des exigences 1 à 3, **caractérisé en ce qu** 'il est construit de manière symétrique par rapport au plan central prévu.

5. Bloc de traitement et / ou de thérapie selon l'une des exigences 1 à 4, **caractérisé en ce qu'**un support latéral (7,8) est agencé le long de l'axe longitudinal (L) de chaque côté des creux (5,6).

6. Bloc de traitement et / ou de thérapie selon l'exigence 5, **caractérisé en ce que** les supports latéraux (7,8) dépassent l'élévation (4) d'une saillie (s), cette saillie (s) étant de préférence comprise entre 0,5 cm et 2 cm.

7. Bloc de traitement et / ou de thérapie selon l'une des exigences 1 à 6, **caractérisé en ce que** l'élévation (4) en direction de l'axe longitudinal (L) présente à son extrémité supérieure une extension (e) comprise entre 0,3 cm et 2 cm.

8. Bloc de traitement et / ou de thérapie selon l'une des exigences 1 à 7, **caractérisé en ce que** l'élévation (4) présente une forme arrondie (9) dans une coupe le long d'un plan comprenant la direction de l'axe longitudinal (L) et la verticale (V), en particulier avec un rayon (r) compris entre 0,15 et 1 cm.
